(19) Europäisches Patentamt
European Patent Office
Office européen des brevets

(11) Publication number: **0 001 689**
**B1**

(12) **EUROPEAN PATENT SPECIFICATION**

(45) Date of publication of patent specification: **21.03.84**

(51) Int. Cl.³: **C 07 D 493/22,** C 07 H 17/08, A 01 N 43/90

(21) Application number: **78300436.9**

(22) Date of filing: **29.09.78**

(54) **Selective hydrogenation products of C-076 compounds, derivatives thereof, their preparation and compositions for the treatment of parasitic infections containing the compounds.**

(30) Priority: **03.10.77 US 838603**

(43) Date of publication of application:
**02.05.79 Bulletin 79/9**

(45) Publication of the grant of the patent:
**21.03.84 Bulletin 84/12**

(84) Designated Contracting States:
**BE CH DE FR GB LU NL SE**

(56) References cited:
**DE - A - 2 329 486**
**DE - A - 2 717 040**
**FR - A - 2 187 778**

**Chemical Abstracts vol. 83, no. 15, 13. October 1975, Columbus, Ohio, USA, A. AOKI et al "Antibiotic B-41A, derivatives from Streptomyces as acaricides" page 163, column 1, abstract no. 127 530 s**

**The file contains technical information submitted after the application was filed and not included in this specification**

(73) Proprietor: **MERCK & CO. INC.**
**126, East Lincoln Avenue P.O. Box 2000**
**Rahway New Jersey 07065 (US)**

(72) Inventor: **Chabala, John Clifford**
**530 Summit Avenue**
**Westfield New Jersey 07090 (US)**
Inventor: **Fisher, Michael Herbert**
**1140 Concord Drive**
**Bridgewater New Jersey 08807 (US)**

(74) Representative: **Crampton, Keith John Allen et al,**
**D YOUNG & CO 10 Staple Inn**
**London WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 001 689 B1**

## Selective hydrogenation products of C—076 compounds, derivatives thereof, their preparation and compositions for the treatment of parasitic infections containing the compounds

This invention relates to C—076.

The term C—076 is used to describe a series of compounds isolated from the fermentation broth of a C—076-producing strain of *Streptomyces avermitilis* as disclosed in our German Published Patent Specification No. 2,717,040. The morphological characteristics of the culture are completely described below. The C—076 compounds are a series of macrolides, each of which is substituted at the 13-position with a 4-(α-L-Oleandrosyl)-α-L-oleandrose group. The 1-series of C—076 compounds also has a 22,23-double bond, as well as several other double bonds. The selective reduction of the 22,23-double bond, without affecting the remaining double bonds is the subject matter of the present application. The C—076 compounds and the present derivatives thereof have a very high degree of anthelmintic and antiparasitic activity.

Summary of the Invention

The C—076 series of compounds have the following structure:

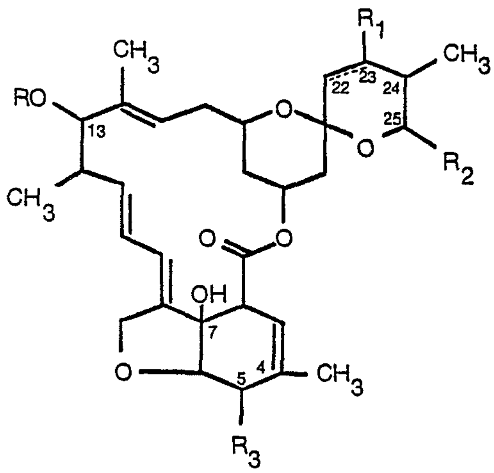

wherein R is the 4'-(α-L-oleandrosyl)-α-L-oleandrose group of the structure:

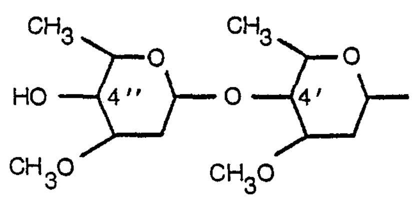

and wherein the broken line indicates that the 22(23) linkage is saturated or ethylenically unsaturated;

$R_1$ is hydroxy and is present only when the 22(23) linkage is saturated;

$R_2$ is *iso*-propyl or *sec*-butyl; and

$R_3$ is methoxy or hydroxy.

There are eight different C—076 compounds and they are given the designations A1a, A1b, A2a, A2b, B1a, B1b, B2a, B2b based upon the structure of the individual compounds.

In the foregoing structural formula, the individual C—076 compounds are as set forth below.

| | $R_1$ | $R_2$ | $R_3$ |
|---|---|---|---|
| A1a | Double bond | *sec*-butyl | $—OCH_3$ |
| A1b | Double bond | *iso*-butyl | $—OCH_3$ |
| A2a | —OH | *sec*-butyl | $—OCH_3$ |
| A2b | —OH | *iso*-propyl | $—OCH_3$ |
| B1a | Double bond | *sec*-butyl | —OH |

| | | | |
|---|---|---|---|
| B1b | Double bond | *iso*-butyl | —OH |
| B2a | —OH | *sec*-butyl | —OH |
| B2b | —OH | *iso*-propyl | —OH |

The C—076 compounds with the 22,23-unsaturations are identified as the "1-series" and it is only these compounds which are reduced to prepare the derivatives of the invention. Either before or after the reduction of the 22,23-double bond further reactions may be carried out in which one or both of the α-L-oleandrose moieties are removed, or in which one or more of the available hydroxy groups are acylated.

Thus, the compounds of the present invention have the following structural formula:

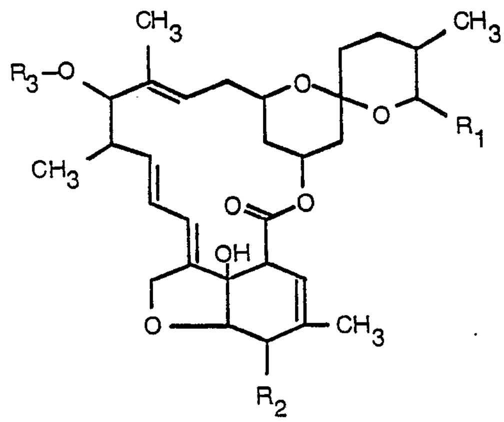

wherein

$R_1$ is *iso*-propyl or sec-butyl;

$R_2$ is methoxy, hydroxy or loweralkanoyloxy;

$R_3$ is hydrogen; loweralkanoyl; α-L-oleandrosyl; 4′-loweralkanoyl-α-L-oleandrosyl; 4′-(α-L-oleandrosyl)-α-L-oleandrosyl; 4″-loweralkanoyl-4′-(α-L-oleandrosyl)-α-L-oleandrosyl.

In the present invention, the term "loweralkanoyl" means those alkanoyl groups of from 2 to 6 carbon atoms such as acetyl, propionyl, butyryl and pivaloyl.

Preferred compounds of the present invention are realized in the above structural formula when:

$R_1$ is *iso*-propyl or *sec*-butyl;

$R_2$ is methoxy or hydroxy; and

$R_3$ is hydrogen, α-L-oleandrosyl or 4′-(α-L-oleandrosyl)-α-L-oleandrosyl.

Additional preferred compounds are realized when the "loweralkanoyl" group of $R_3$ is acetyl in the disaccharide, monosaccharide and aglycone compounds.

As is readily apparent from an analysis of the structure of the C—076 starting materials, there are five unsaturations in the 1-series of compounds.

The present invention involves reducing the 22,23-double bond while not affecting the remaining four unsaturations or any other functional group present on the molecule. It is necessary to select a specific catalyst for the hydrogenation, one that will selectively hydrogenate the least hindered from among a series of unsaturations. The preferred catalyst for such a selective hydrogenation procedure is one having the formula:

$$[(R_4)_3P]_3RhX$$

wherein $R_4$ is $C_{1-6}$ alkyl, phenyl, or ($C_{1-6}$alkyl)-substituted phenyl and X is a halogen.

In the preferred catalyst $R_4$ is phenyl and X is chlorine, that is the compound tris(triphenylphosphine)-rhodium (I) chloride, which is also known as Wilkinson's homogeneous catalyst.

The reaction is carried out using a catalytic amount of the catalyst. The amount of catalyst is not critical and from 0.05 to 0.5 moles of the catalyst for each mole of starting material have been successfully employed. Molar ratios in the range of 0.25 to 0.40 are preferred.

The hydrogenation is carried out in a hydrogen atmosphere which may be either at atmospheric pressure or up to about 4 atmospheres pressure in a standard laboratory hydrogenation apparatus. A solvent is normally employed to dissolve both the starting materials and the catalyst. Preferred solvents are hydrocarbon solvents such as benzene, toluene, petroleum ether and other alkane hydrocarbons. The reaction is complete when the calculated amount of hydrogen has been taken up by the reaction.

This will generally require from about 1 to 48 hours. The reaction may be carried out at from room temperature to about 75°C, however, room temperature is preferred. The hydrogenation products are isolated and purified by techniques known to those skilled in the art.

Other reactions may be carried out on the C—076 starting materials or on the hydrogenated products to prepare the compounds of this invention. While it is possible to complete all of the other reactions on the C—076 starting material and have the hydrogenation step as the final reaction, it is preferred to carry out the hydrogenation step first. Because the 22,23-double bond is somewhat susceptible to nucleophilic addition, reaction conditions for removing the sugar groups or acylating the hydroxy groups must be carefully controlled if the 22,23-double bond is present. If the 22,23-double bond is hydrogenated first, the subsequent sugar removal and acylation is rendered more facile.

Thus, the additional reactions which may be carried out to prepare the compounds of this invention are the selective removal of one or both of the $\alpha$-L-oleandrosyl moieties or the selective acylation of the susceptible hydroxy groups.

The reaction conditions which are generally applicable to the preparation of both the monosaccharide and aglycone involve dissolving the C—076 compound or the hydrogenated C—076 compound in an aqueous acidic non-nucleophilic organic solvent, miscible with water, preferably dioxane, tetrohydrofuran, dimethoxyethane, dimethyl formamide or *bis*-2-methoxyethyl ether, in which the water concentration is from 0.1 to 20% by volume. Concentrated acid is added to the aqueous organic solvent to the extent of 0.01 to 10% by volume. The reaction mixture is generally stirred at 20—40°C, preferably at room temperature, for from 6 to 24 hours. The lower concentrations of acid, from 0.01 to 0.1% will predominately produce the monosaccharide under the above reaction conditions. Higher acid concentrations, from 1 to 10% will predominantly produce the aglycone under the above reaction conditions. Intermediate acid concentrations will generally produce mixtures of monosaccharide and aglycone. The products are isolated, and mixtures are separated by techniques such as column, thin layer preparative and high pressure liquid chromatography, and other known techniques.

The acids which may be employed in the above process include mineral acids and organic acids such as sulfuric, hydrohalic, phosphoric, trifluoroacetic and trifluoromethane sulfonic acids. The hydrohalic acids are preferably hydrochloric or hydrobromic. The preferred acid in the above process is sulfuric acid.

A further procedure for the preparation of the monosaccharide or aglycone of the C—076 compounds or of the hydrogenated C—076 compounds utilizes a different solvent system for the monosachharide and the aglycone. The procedure for the preparation of the monosaccharide uses 1% acid by volume in isopropanol at from 20—40°C, preferably room temperature, for from 6 to 24 hours. For the preparation of the aglycone, 1% acid, by volume, in methanol under the foregoing reaction conditions has been found to be appropriate.

When this procedure is employed on the starting material (the compounds with the 22,23-double bond) there is a possibility of nucleophilic addition to the double bond. If such occurs, chromatographic purification will remove the by-product in order to allow for further reactions.

The acids listed above are appropriate for this process, and again sulfuric acid is the preferred acid.

The above described compounds are isolated from the reaction mixture and mixtures of compounds are separated using techniques known to those skilled in this art, and in particular the chromatographic techniques described above.

The acylated compounds are prepared using acylation techniques in which the reaction conditions will vary, depending upon the reactivity of the hydroxy group being acylated. Where there is more than one hydroxy group to be acylated, different reaction conditions are employed to minimize the formation of mixtures.

The acylation reagents employed are generally the halide, preferably the chloride, of the above loweralkanoyl groups. That is the loweralkanoyl halide reagent is generally employed.

In addition, the acylation reagent could be in the form of the anhydride or of the halo formate. In the case of reactions carried out with the halide reagents, it is often advantageous to include in the reaction mixture a basic compound capable of reacting with and neutralizing the hydrogen halide which is liberated during the course of the reaction. Tertiary amines are preferred such as triethylamine, pyridine, dimethylamino pyridine and diisopropyl ethylamine. The basic compound is required in equimolar amounts relative to the numbered moles of hydrogen halide being liberated, however excess amounts, even using the basic compound as a solvent, are not detrimental.

In the case of the A1 compounds of C—076, or of the hydrogenated C—076 Ā1 compounds there is only a single hydroxy group, 4″ hydroxy, which may be acylated. The formation of the monosaccharide or the aglycone still leaves only a single hydroxy group which may be acylated, that is the 4′ or 13 hydroxy group.

In the case of the 4″, 4′ and 13 hydroxy groups of C—076 A1 compounds, the acylating reagent is dissolved in a suitable solvent, pyridine is preferred, and the acylating reagent added. The reaction is maintained at from 0°C to room temperature for from 4 to 24 hours. The product is isolated using known techniques.

The B1 compounds have 2 available hydroxy groups: at the 4″ (4′ or 13) and the 5-positions. However, the two hydroxy groups have similar reactivities. When the reaction of the acylating agent in pyridine is carried out at about room temperature for from 4 to 24 hours, the diacyl compound is recovered. When the reaction is carried out at 0°C a mixture of the 4″ (4′ or 13) and 5 monoacyl compounds are recovered. To recover individual compounds, the mxiture is placed on a chromatographic column or a preparative layer chromatographic plate of alumina or silica gel and the individual compounds are readily isolated. In addition, techniques such as high pressure liquid chromatography may be employed to separate mixtures of acylated compounds.

The acyl compounds thus prepared are isolated from the reaction mixture using techniques known to those skilled in this art.

The novel compounds of this invention have significant parasiticidal activity as anthelmintics, ectoparasiticides, insecticides and acaricides, in human and animal health and in agriculture.

The disease or group of diseases described generally as helminthiasis is due to infection of an animal host with parasitic worms known as helminths. Helminthiasis is a prevalent and serious economic problem in domesticated animals such as swine, sheep, horses, cattle, goats, dogs, cats and poultry. Among the helminths, the group of worms described as nematodes causes widespread and often times serious infection in various species of animals. The most common genera of nematodes infecting the animals referred to above are *Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris* and *Parascaris.* Certain of these, such as *Nematodirus, Cooperia,* and *Oesphagostomum* attack primarily the intestinal tract while others, such as *Haemonchus* and *Ostertagia*, are more prevalent in the stomach while still others such as *Dictyocaulus* are found in the lungs. Still other parasites may be located in other tissues and organs of the body such as the heart and blood vessels, subcutaneous and lymphatic tissue and the like. The parasitic infections known as helminthiases lead to anemia, malnutrition, weakness, weight loss, severe damage to the walls of the intestinal tract and other tissues and organs and, if left untreated, may result in death of the infected host. The hydrogenated C—076 compounds of this invention have unexpectedly high activity against these parasites, and in addition are also active against *Dirofilaria* in dogs, *Nematospiroides, Syphacia, Aspiculuris* in rodents, arthropod ectoparasites of animals and birds such as ticks, mites, lice, fleas, blowfly, in sheep *Lucilia Sp.,* biting insects and such migrating diperous larvae as *Hypoderma sp.* cattle, *Gastrophilus* in horses, and *Cuterebra sp.* in rodents.

The compounds of the present invention are also useful against parasites which infect humans. The most common genera of parasites of the gastro-intestinal tract of man are *Ancylostoma, Necator, Ascaris, Strongyloides, Trichinella, Capillaria, Trichuris,* and *Enterobius.* Other medically important genera of parasites which are found in the blood or other tissues and organs outside the gastro-intestinal tract are the filiarial worms such as *Wuchereria, Brugia, Onchocerca* and *Loa, Dracunculus* and extra intestinal stages of the intestinal worms *Strongyloides* and *Trichinella.* The compounds are also of value against arthropods parasitizing man, biting insects and other dipterous pests causing annoyance to man.

The compounds are also active against household pests such as the cockroach, *Blatella sp.,* clothes moth, *Tineola sp.,* carpet beetle, *Attagenus sp.,* and the housefly *Musca domestica.*

The compounds are also useful against insect pests of stored grains such as *Tribolium sp., Tenebrio sp.*, and of agricultural plants such as spider mites, (*Tetranychus sp.*), aphids, (*Acyrthiosiphon sp.)*; against migratory orthopterans such as locusts and immature stages of insects living on plant tissue. The compounds are useful as a nematocide for the control of soil nematodes and plant parasites such as *Meloidogyne spp.* which may be of importance in agriculture.

These compounds may be administered orally in a unit dosage form such as a capsule, bolus or tablet, or as a liquid drench where used as an anthelmintic in mammals. The drench is normally a solution, suspension or dispersion of the active ingredient usually in water together with a suspending agent such as bentonite and a wetting agent or like excipient. Generally, the drenches also contain an antifoaming agent. Drench formulations generally contains from 0.001 to 0.5% by weight of the active compound. Preferred drench formulations may contain from 0.01 to 0.1% by weight. The capsules and boluses comprise the active ingredient admixed with a carrier vehicle such as starch, talc, magnesium stearate, or di-calcium phosphate.

Where it is desired to administer the C—076 derivatives in a dry, solid unit dosage form, capsules, boluses or tablets containing the desired amount of active compound usually are employed. These dosage forms are prepared by intimately and uniformly mixing the active ingredient with suitable finely divided diluents, fillers, disintegrating agents and/or binders such as starch, lactose, talc, magnesium stearate and vegetable gums. Such unit dosage formulations may be varied widely with respect to their total weight and content of the antiparasitic agent depending upon factors such as the type of host animal to be treated, the severity and type of infection and the weight of the host.

When the active compound is to be administered via an animal feedstuff, it is intimately dispersed in the feed or used as a top dressing or in the form of pellets which may then be added to the finished feed or optionally fed separately. Alternatively, the antiparasitic compounds of our invention may be

administered to animals parenterally, for example, by intraruminal, intramuscular, intratracheal, or subcutaneous injection in which event the active ingredient is dissolved or dispersed in a liquid carrier vehicle. For parenteral administration, the active material is suitably admixed with an acceptable vehicle, preferably of the vegetable oil variety such as peanut oil or cotton seed oil. Other parenteral vehicles such as organic preparation using solketal, glycerol formal, and aqueous parenteral formulations are also used. The active monosaccharide or aglycone C—076 compound or compounds are dissolved or suspended in the parenteral formulation for administration; such formulations generally contain from 0.005 to 5% by weight of the active compound.

Although the antiparasitic agents of this invention find their primary use in the treatment and/or prevention of helminthiasis, they are also useful in the prevention and treatment of diseases caused by other parasites, for example, arthropod parasites such as ticks, lice, fleas, mites and other biting insects in domesticated animals and poultry. They are also effective in treatment of parasitic diseases that occur in other animals including humans. The optimum amount to be employed for best results will, of course, depend upon the particular compound employed, the species of animal to be treated and the type and severity of parasitic infection or infestation. Generally good results are obtained with our novel compounds by the oral administration of from 0.001 to 10 mg. per kg. of animal body weight, such total dose being given at one time or in divided doses over a relatively short period of time such as 1—5 days. With the preferred compounds of the invention, excellent control of such parasites is obtained in animals by administering from 0.025 to 0.5 mg. per kg. of body weight in a single dose. Repeat treatments are given as required to combat re-infections and are dependent upon the species of parasite and the husbandry techniques being employed. The techniques for administering these materials to animals are known to those skilled in the veterinary field.

When the compounds described herein are administered as a component of the feed of the animals, or dissolved or suspended in the drinking water, compositions are provided in which the active compound or compounds are intimately dispersed in an inert carrier or diluent. By inert carrier is meant one that will not react with the antiparasitic agent and one that may be administered safely to animals. Preferably, a carrier for feed administration is one that is, or may be, an ingredient of the animal ration.

Suitable compositions include feed premixes or supplements in which the active ingredient is present in relatively large amounts and which are suitable for direct feeding to the animal or for addition to the feed either directly or after an intermediate dilution or blending step. Typical carriers or diluents suitable for such compositions include, for example, distillers' dried grains, corn meals, citrus meal, fermentation residues, ground oyster shells, wheat shorts, molasses solubles, corn cob meal, edible bean mill feed, soya grits and crushed limestone. The active hydrogenated C—076 compounds are intimately dispersed throughout the carrier by methods such as grinding, stirring, milling or tumbling. Compositions containing from 0.005 to 2.0% by weight of the active compound are particularly suitable as feed premixes. Feed supplements, which are fed directly to the animal, contain from 0.0002 to 0.3% by weight of the active compounds.

Such supplements are added to the animal feed in an amount to give the finished feed the concentration of active compound desired for the treatment and control of parasitic diseases. Although the desired concentration of active compound will vary depending upon the factors previously mentioned as well as upon the particular C—076 derivative employed, the compounds of this invention are usually fed at concentrations of between 0.00001 to 0.002% in the feed in order to achieve the desired antiparasitic result.

In using the compounds of this invention, the individual hydrogenated C—076 components may be prepared and used in that form. Alternatively, mixtures of two or more of the individual hydrogenated C—076 components may be used, as well as mixtures of the parent C—076 compounds other C—076 compound or other active compounds not related to C—076 and the compounds of this invention.

In the isolation of the C—076 compounds, which serve as starting materials for the processes of the present invention, from the fermentation broth, the various C—076 compounds will be found to have been prepared in unequal amounts. In particular an "a" series compound will be prepared in a higher proportion than the corresponding "b" series compound. The weight ratio of "a" series to the corresponding "b" series is 75:25 to 99:1. The differences between the "a" series and "b" series is constant throughout the C—076 compounds and consists of a *sec*-butyl group and an *iso*-propyl group respectively at the 25 position. This difference, of course, does not interfere with any of the present reactions. In particular it may not be necessary to separate the "b" components from the related "a" component. Separation of these closely related compounds is generally not practiced since the "b" compound is present only in a very small percent by weight, and the structural difference has negligible effect on the reaction processes and biological activities.

In particular it has been found that the starting materials for the compounds of this invention are very often prepared in a ratio of 80% C—076 B1a or A1a and 20% C—076 B1b or A1b. Thus the preferred composition of this invention is one which contains about 80% of the "a" component and 20% of the "b" component.

The C—076 compounds of this invention are also useful in combatting agricultural pests that inflict damage upon crops while they are growing or while in storage. The compounds are applied using

known techniques such as sprays, dusts and emulsions to the growing or stored crops to effect protection from such agricultural pests.

The following examples are provided in order that this invention might be more fully understood; they are not to be construed as limitative of the invention.

The hydrogenated C—076 derivatives prepared in the following examples are generally isolated as amorphous solids and not as crystalline solids. They are thus characterized analytically using techniques such as mass spectrometry and nuclear magnetic resonance. Being amorphous, the compounds are not characterized by sharp melting points, however, the chromatographic and analytical methods employed indicate that the compounds are pure.

Example 1

*22,23-Dihydro C—076 A1a*

51.0 mg of C—076 A1a and 14.4 mg. of tris (triphenylphosphine) rhodium (I) chloride are combined in 3.5 ml. of benzene and hydrogenated for 20 hours at room temperature under atmospheric pressure. The crude reaction mixture is chromatographed on a preparative layer chromatography plate eluting twice with 10% tetrahydrofuran in chloroform. The product is removed from the support using ethyl acetate which is evaporated to dryness and the residue analyzed with 300 MHz nuclear magnetic resonance and mass spectroscopy indicating the preparation of 22,23-dihydro C—076 A1a.

Example 2

*22,23-Dihydro C—076 B1a*

The solution of 87.3 mg. of C—076 B1a in 6 ml. of benzene containing 25 mg. of tris (triphenylphosphine) rhodium (I) chloride is hydrogenated for 4 hours at room temperature under 1 atmosphere of hydrogen pressure. Preparative layer chromatography on silica gel eluting with 20% tetrahydrofuran in chloroform recovers starting material. The sample is rehydrogenated following the above conditions for 19 hours. Preparative layer chromatography recovers 55 mg. of 22,23-dihydro C—076 B1a which is identified by mass spectrometry and 300 MHz nuclear magnetic resonance.

Example 3

*22,23-Dihydro C—076 B1a*

A solution of 1.007 g. of C—076 B1a 314 mg. of tris (triphenylphosphine) rhodium (I) chloride and 33 ml. of benzene is hydrogenated for 21 hours at room temperature under 1 atmosphere of hydrogen pressure. The solvent is removed *in vacuo* and the residue dissolved in a 1:1 mixture of methylene chloride and ethyl acetate and filtered. The filtrate is placed on a column of 60 g. of silica gel eluting with a 1:1 mixture of methylene chloride and ethyl acetate taking 10 ml. fractions. Fractions 14—65 are combined and evaporated to dryness affording 1.118 g. of a solid material which is indicated by high pressure liquid chromatography to be a 60/40 mixture of the hydrogenated product and starting material. The mixture is rehydrogenated in 55 ml. of benzene adding 310 mg. of tris (triphenylphosphine) rhodium (I) chloride and stirring for 21 hours at room temperature under 1 atmosphere of hydrogen pressure. The solvent is removed in vacuo and the residue chromatographed on 80 g. of silica gel using 40:60 mixture of ethyl acetate and methylene chloride as eluant. 10 ml. fractions are taken and the product appears in fractions 26—80. These fractions are combined and evaporated to dryness *in vacuo* affording a yellow oil. The oil is dissolved in benzene and lyophilized affording a pale yellow powder which is identified as 22,23-dihydro C—076 B1a by mass spectrometry and 300 MHz nuclear magnetic resonance. 0.976 G. of product is obtained.

Example 4

*22,23-Dihydro C—076 A1a Monosaccharide*

11.2 mg. of 22,23-dihydro C—076 A1a is dissolved in 1.1 ml. of 1% sulfuric acid in isopropanol and stirred for 20 hours at room temperature. The reaction mixture is diluted with chloroform to a volume of about 5.0 ml. and washed with saturated aqueous sodium bicarbonate solution and sodium chloride solution. The organic layer is dried over sodium sulfate and evaporated to dryness *in vacuo* affording an oil. The oil is placed on a silica gel preparative layer chromatography plate and eluted with 5% tetrahydrofuran in chloroform. The product is removed from the plate and lyophilized from benzene affording 5.2 mg. of a white powder which is identified by 300 MHz nuclear magnetic resonance and mass spectrometry as 22,23-dihydro C—076 A1a monosaccharide.

Example 5

*22,23-Dihydro C—076 A1a Aglycone*

10.1 mg. of 22,23-dihydro C—076 A1a is stirred for 20 hours in 1.1 ml. of 1% sulfuric acid in methanol at room temperature. The reaction mixture is treated as in Example 4 affording an oil which is purified by preparative layer chromatography on silica gel eluting with % tetrahydrofuran in chloroform. The product is removed from the chromatography plate and lyophilized from benzene affording 4.2 mg. of a white powder which 300 MHz nuclear magnetic resonance and mass spectrometry indicate to be 22,23-dihydro C—076 A1a aglycone.

Example 6

*22,23-Dihydro C—076 B1a Monosaccharide*

395 mg. of 22,23-dihydro C—076 B1a is added to a stirred solution of 50 ml. of 1% sulfuric acid in isopropanol and the solution is stirred for 14 hours at room temperature. The reaction mixture is treated as in Example 4 affording 0.404 g. of a foam after lyophilization from benzene. The foam is chromatographed on 6 preparative layer silica gel chromatography plates eluting twice with 4% tetrahydrofuran in chloroform. The monosaccharide with a Rf 0.15 is collected and washed from the silica gel with a total of 650 ml. of ethyl acetate. The combined washings are evaporated to dryness and the residue lyophilized from benzene to afford 0.2308 g. of 22,23-dihydro C—076 B1a monsaccharide which high pressure liquid chromatography indicates to be essentially pure.

Example 7

*22,23-Dihydro C—076 B1a Aglycone*

9.7 mg. of 22,23-dihydro C—076 B1a is stirred overnight in 1 ml. of a 1% sulfuric acid in methanol solution. The reaction mixture is treated as in Example 4 and the solid material treated with preparative layer chromatography on silica gel eluting with 10% tetrahydrofuran in chloroform. The oil recovered from the chromatography plate is lyophilized from benzene affording 4.7 mg. of a white powder which 300 MHz nuclear magnetic resonance and mass spectrometry indicate to be 22,23-dihydro C—076 B1a aglycone.

Example 8

*22,23-Dihydro C—076 B1a Aglycone*

0.486 g. of 22,23-dihydro C—076 B1a is added to a stirred solution of 50 ml. of 1% sulfuric acid in methanol and the reaction mixture stirred for 13 hours at room temperature. The reaction mixture is diluted with 250 ml. of methylene chloride and washed with 50 ml. of saturated aqueous potassium bicarbonate and 50 ml. of water. The aqueous layer is washed twice with 20 ml. portions of methylene chloride and the combined organic phases are dried with saturated brine and sodium sulfate and evaporated to dryness *in vacuo* affording 0.480 g. of a pale yellow foam. The foam is dissovled in 4 ml. of methylene chloride and placed on 4 preparative layer chromatography silica gel plates and eluted 4 times with 4% tetrahydrofuran and chloroform. The product is recovered from the silica gel plates affording an oily residue which is lyophilized from benzene affording 255.8 mg. of a white solid. Traces of methyl oleandroside are indicated to be present in the solid material. The white solid is then lyophilized again from benzene and placed under high vacuum for 20 hours to remove the impurity affording 22,23-dihydro C—076 B1a aglycone.

Example 9

*4″-O-acetyl-22,23-Dihydro C—076 A1a*

6.8 mg. of 22,23-dihydro C—076 A1a is dissolved in 40 drops of anhydrous pyridine, chilled to 0°C and treated with 20 drops of acetic anhydride. The reaction mixture is allowed to warm to room temperature and stirred overnight. The reaction mixture is diluted with 5 ml. of ether and 6 ml. of water and the layers separated. The aqueous phase is washed twice with ether and the organic layers combined and back washed 3 times with water. The ether layer is dried over magnesium sulfate and evaporated to dryness *in vacuo* affording an oil. The oil is chromatographed on silica gel preparative layer chromatography plates eluting with 5% tetrahydrofuran in chloroform. The product is recovered from the plates and lyophilized from benzene affording 6.1 mg. of 4″-O-acetyl-22,23-dihydro C—076 A1a as determined by mass spectrometry at 300 MHz nuclear magnetic resonance.

Example 10

*4″-O-acetyl-22,23-Dihydro C—076 B1a and 4″,5-di-O-acetyl 22,23-Dihydro C—076 B1a*

18.6 mg. of 22,23-dihydro C—076 B1a is dissolved in 63 drops (about 1 ml.) of dry pyridine and treated with 9 drops of acetic anhydride at 0°C. The reaction is stirred under nitrogen for 6 hours at 0°C. The mixture is then quenched with 5 ml. of water and extracted 3 times with 3 ml. portions of ether. The combined ether extracts are then washed 3 times with 3 ml. portions of water and dried over magnesium sulfate and evaporated to dryness *in vacuo.* The oil is chromatographed on preparative layer silica gel chromatography plates eluting twice with 5% tetrahydrofuran in chloroform affording 5.8 mg. of 4″-O-acetyl-22,23-dihydro C—076 B1a and 5.8 mg. of 4″,5-di-O-acetyl-22,23-dihydro C—076 B1a after lyophilization from benzene. The structures are confirmed by 300 MHz nuclear magnetic resonance and mass spectrometry.

Example 11

*22,23-Dihydro C—076 B1a*

39 g. of C—076 B1a is dissolved in 1540 ml. of toluene and introduced into a 4 liter stirred autoclave. To this is added 3.9 g. of tris(triphenylphosphine)rhodium(I) chloride (Wilkinson's catalyst). A hydrogenation pressure of 40 psi. and a temperature of 40°C is maintained with stirring for 4 1/2 hours. At the end of this period liquid chromatographic analysis indicates 98% yield of dihydro C—076

B1a with 1.5% of tetrahydro C—076 B1a. The toluene is removed by evaporation *in vacuo* and the dark red gum is dissolved in ethanol at a rate of 4 ml. of ethanol per gram of product. Formamide at a rate of 10 ml. per gram of product is added and the solution heated on the steam bath to 40—50° while water is added at a rate of 2 ml. per gram of product. After crystallization commences the heat is removed and the solution allowed to cool slowly with stirring overnight. The solid is filtered off and washed with a mixture 3 parts water and 1 part ethanol and dried *in vacuo* overnight. The solids are dissolved in 150 ml. of ethanol and warmed to 35—40°C on the steam bath. Water, 150 ml. is added slowly with stirring. When solution is complete at 35°C the heat is removed and the solution allowed to cool slowly overnight. The crystals are removed by filtration and washed with 50% aqueous ethanol and dried *in vacuo* overnight affording 32.55 g. of 22,23-dihydro C—076 B1a with m.p. of 155—157°C.

Based on taxonomic studies, the microorganisms capable of producing C—076 compounds are of a new species of the genus Streptomyces, which has been named *Streptomyces avermitilis*. One such culture, isolated from soil, is designated MA—4680 in the culture collection of Merck & Co. Inc., Rahway, New Jersey. A C—076-producing sample of this culture has been deposited in the permanent culture collection of the Fermentation Section of the Northern Utilization Research Branch, U.S. Department of Agriculture at Peoria, Illinois, and has been assigned the accession number NRRL 8165. A sample of NRRL 8165 has also been deposited, without restriction as to availability, in the permanent culture collection of the American Type Culture Collection at 12301 Parklawn Drive, Rockville, Maryland 20852, and has been assigned the accession number ATCC 31,267.

The morphological and cultural characteristics of *Streptomyces avermitilis* are set forth below:

Morphology: Sporophores form spirals as side branches on aerial mycelia. Spirals are compact but become more open as culture ages. Spores are in chains of more than 15 spores and are usually spherical to oval at 970 X magnification. Sporulation is observed on oatmeal agar, glycerol-asparagine agar, salts-starch agar and egg albumin agar. Spore surface is smooth as seen be electron microscopy.

| | | |
|---|---|---|
| Oatmeal agar | | |
| | Vegetative growth: | Reverse — very dark brown |
| | Aerial mycelium: | Powdery, brownish gray (41i)* mixed with white. |
| | Soluble pigment: | Brown |
| Czapek Dox agar (sucrose nitrate agar) | | |
| | Vegetative growth: | Poor, colorless |
| | Aerial mycelium: | Scant, grayish |
| | Soluble pigment: | Light grayish tan |
| Egg albumin agar | | |
| | Vegetative growth: | Tan |
| | Aerial mycelium: | Moderate, light grayish-yellow-brown (3ge)* mixed with white. |
| | Soluble pigment: | Light yellowish tan |
| Glycerol asparagine agar | | |
| | Vegetative growth: | Reverse — yellowish brown |
| | Aerial mycelium: | Powdery, brownish gray (41i)* mixed with white. |
| | Soluble pigment: | Light, yellowish brown |
| Inorganic salts-starch agar | | |
| | Vegetative growth: | Reverse — grayish yellowish brown. |
| | Aerial mycelium: | Powdery, light brownish gray (4ig)* edged with darker brownish gray (4li).* |
| | Soluble pigment: | Light yellowish brown |
| Yeast extract-dextrose + salts agar | | |
| | Vegetative growth: | Reverse — dark brown |
| | Aerial mycelium: | Moderate, brownish white |
| | Soluble pigment: | Brown |
| Yeast extract-malt extract agar | | |
| | Vegetative growth: | Reverse — dark brown |
| | Aerial mycelium: | Moderate, brownish white |
| | Soluble pigment: | Brown |

Peptone-iron-yeast extract agar

| | |
|---|---|
| Vegetative growth: | Dark brown |
| Aerial Mycelium: | None |
| Soluble pigment: | Dark brown to black |
| Melanin: | Positive |
| H S production | Positive |

Nutrient agar

| | |
|---|---|
| Vegetative growth: | Tan |
| Aerial mycelium: | Sparse, grayish |
| Soluble pigment: | Light brown |

Nutrient starch agar

| | |
|---|---|
| Vegetative growth: | Tan |
| Aerial mycelium: | Sparse, grayish white |
| Soluble pigment: | Light brown |
| Hydrolysis of starch: | Good |

Potato plug

| | |
|---|---|
| Vegetative growth: | Tan |
| Aerial mycelium: | Brown mixed with grayish white |
| Soluble pigment: | Grayish brown |

Loeffler's Blood serum

| | |
|---|---|
| Vegetative growth: | Grayish tan |
| Aerial mycelium: | None |
| Soluble pigment: | Some browning of medium |
| Liquefaction: | None |

Nutrient tyrosine agar:

| | |
|---|---|
| Vegetative growth: | Reverse — dark brown to black |
| Aerial mycelium: | Sparse, grayish |
| Soluble pigment: | Dark brown |
| Decomposition of tyrosine: | None |

Carbon utilization

Pridham-Gottlieb basal medium + 1% carbon source; + = growth; no growth as compared to negative control (no carbon source).

| | |
|---|---|
| Glucose | + |
| Arabinose | + |
| Cellulose | – |
| Fructose | + |
| Inositol | + |
| Lactose | + |
| Maltose | + |
| Mannitol | + |
| Mannose | + |
| Raffinose | + |
| Rhamnose | + |
| Sucrose | + |
| Xylose | + |

Nutrient gelatin agar

| | |
|---|---|
| Vegetative growth: | Tan |
| Aerial mycelium: | Sparse, grayish white |
| Soluble pigment: | Light brown |
| Liquefaction of gelatin: | Good |

Gelatin stabs

| | |
|---|---|
| Vegetative growth: | Brown ring |
| Aerial mycelium: | None |
| Soluble pigment: | Greenish brown |
| Liquefaction of gelatin: | Complete |

Skim milk agar

| | | |
|---|---|---|
| | Vegetative growth: | Dark brown |
| | Aerial mycelium: | None |
| | Soluble pigment: | Dark brown |
| | Hydrolysis of casein: | Good |

Litmus milk

| | | |
|---|---|---|
| | Vegetative growth: | Dark brown growth ring |
| | Aerial mycelium: | None |
| | Color: | Dark brown |
| | Coagulation and/or peptonization: | Complete peptonization; becoming alkaline (pH 8.1). |

Skim milk

| | | |
|---|---|---|
| | Vegetative growth: | Dark brown growth ring |
| | Aerial mycelium: | None |
| | Soluble pigment: | Dark brown |
| | Coagulation and/or peptonization: | Complete peptonization; becoming alkaline (pH 8.0). |

Temperature range: (Yeast extract-dextrose + salts agar)
28°C — Good vegetative growth and aerial mycelia
37°C — Good vegetative growth and aerial mycelia
50°C — No growth

Oxygen requirement: (Stab culture in yeast extract-dextrose + salts agar)
Aerobic

All readings taken after three weeks at 28°C unless noted otherwise. pH of all media approximately neutral (6.8—7.2) Color number designations (*) taken from Color Harmony Manual, 1958, 4th Edition Container Corporation of America, Chicago, Illinois.

A careful comparison of the foregoing data with published descriptions including Bergey's Manual of Determinative Bacteriology (Eighth Edition) of known microorganisms reveals significant differences that indicate that the microorganism should be classified as a new species. On this basis, it was designed *Streptomyces avermitilis.*

Other organisms can also be used to produce C—076, e.g. mutants obtained by mutating agents such as X-ray irradiation, ultraviolet irradiation or nitrogen mustards.

A culture of one such organism was isolated after irradiating *S. avermitilis* with ultraviolet light. A lyophilized tube and a frozen vial of this culture have been deposited in the permanent culture collection of the American Type Culture Collection, and they have been assigned the accession numbers 31272 and 31271 respectively. Slightly higher fermentation yields of C—076 have been obtained using this frozen stock as inoculum.

## Claims

1. A compound having the formula:

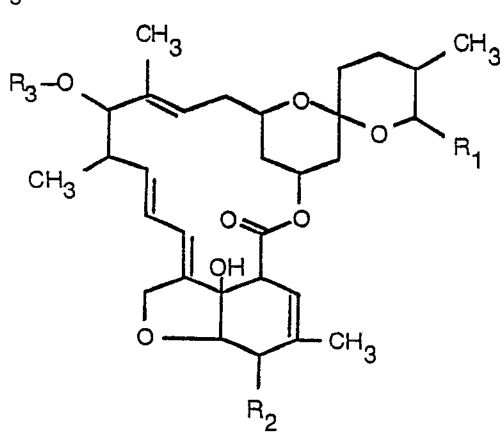

in which $R_1$ is *iso*-propyl or *sec*-butyl; $R_2$ is methoxy, hydroxy or loweralkanoyloxy; and $R_3$ is hydrogen;

lower alkanoyl; α-*L*-oleandrosyl; 4'-(lower alkanoyl)-α-*L*-oleandrosyl; 4'-(α-*L*-oleandrosyl)-α-*L*-oleandrosyl or 4''-(lower alkanoyl)-4'-(α-*L*-oleandrosyl)-α-*L*-oleandrosyl, where "lower alkanoyl" means an alkanoyl group containing from 2 to 6 carbon atoms.

2. A compound as claimed in Claim 1 in which $R_1$ is *iso*-propyl; $R_2$ is methoxy or hydroxy; and $R_3$ is hydrogen, α-*L*-oleandrosyl or 4'-(α-*L*-oleandrosyl)-α-*L*-oleandrosyl.

3. A compound as claimed in Claim 1 in which $R_1$ is secbutyl and $R_2$ and $R_3$ are as defined in Claim 2.

4. 22,23-Dihydro-C—076 B1a, the compound claimed in Claim 1 in which $R_1$ is sec butyl, $R_2$ is hydroxy and $R_3$ is hydrogen.

5. 22,23-Dihydro-C—076 B1a monosaccharide, the compounds claimed in Claim 1 in which $R_1$ is sec. butyl, $R_2$ is hydroxy and $R_3$ is α-*L*-oleandrosyl.

6. A method of preparing a compound as claimed in Claim 1 that comprises treating a compound having the formula:

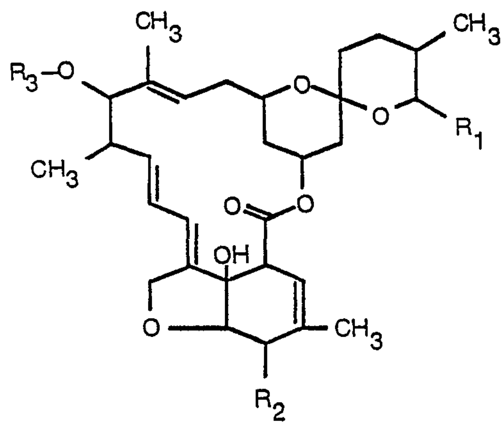

in which $R_1$, $R_2$ and $R_3$ are as defined in Claim 1, with hydrogen in the presence of a catalytic amount of a compound having the formula $[(R_4)_3P]_3RhX$ in which $R_4$ is a $C_{1-6}$ alkyl, phenyl or ($C_{1-6}$ alkyl)-substituted phenyl radical and X is a halogen atom.

7. A compound as claimed in Claim 1 or a mixture of two or more such compounds for use as a parasiticide.

8. A composition for the treatment of parasitic infections that comprises an inert carrier and one or more compounds as claimed in Claim 1.

9. A composition as claimed in Claim 8 in which the active ingredient is a mixture of about 80% 22,23-dihydro C—076 B1a as defined in Claim 4 and, correspondingly, about 20% 22,23-dihydro C—076 B1b, which is the compound claimed in Claim 1 in which $R_1$ is isopropyl, $R_2$ is hydroxy and $R_3$ is hydrogen.

**Revendications**

1. Un composé répondant à la formule:

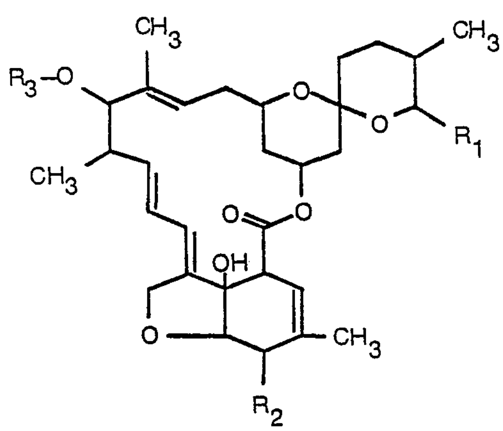

dans laquelle $R_1$ est un isopropyle ou un sec-butyle; $R_2$ est un méthoxy, un hydroxy ou un alcanoyloxy inférieur; et $R_3$ est un hydrogène; un alcanoyle inférieur; un α-L-oléandrosyle; un 4'-(alcanoyl inférieur)-α-L-oléandrosyle; un 4'-(α-L-oléandrosyl)-α-L-oléandrosyle ou un 4''-(alcanoyl inférieur)-4'-(α-L-oléandrosyl)-α-L-oléandrosyle, où "alcanoyle inférieur" désigne un groupe alcanoyle contenant 2 à 6 atomes de carbone.

2. Un composé comme revendiqué dans la revendication 1, dans lequel $R_1$ est un isopropyle; $R_2$ est un méthoxy ou un hydroxy; et $R_3$ est un hydrogène, un α-*L*-oléandrosyle ou un 4'-(α-*L*-oléandrosyl)-α-*L*-oléandrosyle.

3. Un composé comme revendiqué dans la revendication 1, dans lequel $R_1$ est un sec-butyle et $R_2$ et $R_3$ sont comme défini dans la revendication 2.

4. 22,23-dihydro-C—076 B1a, le composé revendiqué dans la revendication 1, dans lequel $R_1$ est un sec-butyle, $R_2$ est un hydroxy et $R_3$ est un hydrogène.

5. Monosaccharide de 22,23-dihydro-C—076 B1a, le composé revendiqué dans la revendication 1, où $R_1$ est un sec-butyle, $R_2$ est un hydroxy et $R_3$ est un α-*L*-oléandrosyle.

6. Un procédé pour préparer un composé comme revendiqué dans la revendication 1, qui comprend le traitement d'un composé ayant pour formule:

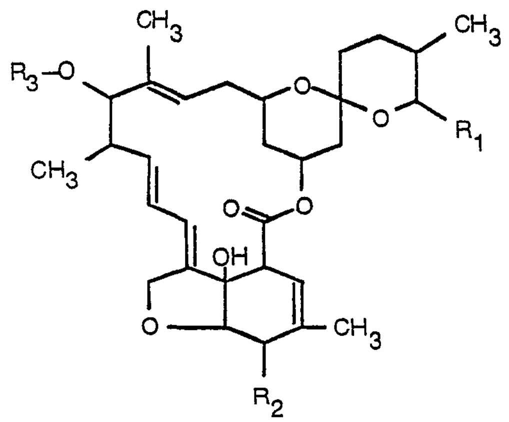

dans laquelle $R_1$, $R_2$ et $R_3$ sont comme défini dans la revendication 1, avec de l'hydrogène en présence d'une quantité catalytique d'un composé ayant pour formule $[(R_4)_3P]_3RhX$ dans laquelle $R_4$ est un radical alkyle en $C_{1-6}$, phényle ou phényle substitué par un alkyle en $C_{1-6}$, et, X est un atome d'halogène.

7. Un composé comme revendiqué dans la revendication 1 ou un mélange de deux ou plusieurs de tels composés pour l'emploi comme parasiticide.

8. Une composition pour le traitement des infections parasitaires qui comprend un support inerte et un ou plusieurs composés comme revendiqué dans la revendication 1.

9. Une composition comme revendiqué dans la revendication 8, dans laquelle l'ingrédient actif est un mélange d'environ 80% de 22,23-dihydro C—076 B1a comme défini dans la revendication 4, et de façon correspondante, d'environ 20% de 22,23-dihydro C—076 B1b, qui est le composé revendiqué dans la revendication 1, où $R_1$ est un isopropyle, $R_2$ est un hydroxy et $R_3$ est un hydrogène.

**Patentansprüche**

1. Verbindung mit der Formel

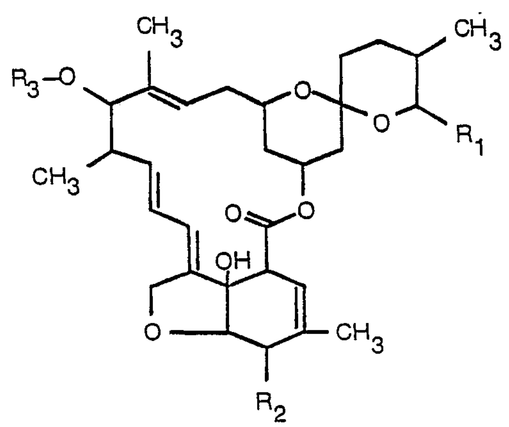

worin $R_1$ Isopropyl oder sec-Butyl ist; $R_2$ Methoxy, Hydroxy oder niedrig-Alkanoyloxy ist; und $R_3$ Wasserstoff, niedrig-Alkanoyl; α-*L*-Oleandrosyl; 4'-(niedrig-Alkanoyl)-α-*L*-oleandrosyl; 4'-(α-*L*-Oleandrosyl)-α-*L*-oleandrosyl oder 4''-(niedrig-Alkanoyl)-4'-(α-*L*-oleandrosyl)-α-*L*-oleandrosyl ist, worin "niedrig-Alkanoyl" eine Alkanoylgruppe mit 2 bis 6 Kohlenstoffatomen bedeutet.

2. Verbindung nach Anspruch 1, worin $R_1$ Isopropyl ist; $R_2$ Methoxy oder Hydroxy ist; und $R_3$ Wasserstoff, *α-L*-Oleandrosyl oder 4'-(*α-L*-Oleandrosyl)-*α-L*-oleandrosyl ist.

3. Verbindung nach Anspruch 1, worin $R_1$ sec.-Butyl ist und $R_2$ und $R_3$ wie in Anspruch 2 definiert sind.

4. 22,23-Dihydro-C—076 B1a, die Verbindung nach Anspruch 1, worin $R_1$ sec-Butyl ist, $R_2$ Hydroxy ist und $R_3$ Wasserstoff ist.

5. 22,23-Dihydro-C—076 B1a-monosaccharid, die Verbindungen nach Anspruch 1, worin $R_1$ sec-Butyl ist, $R_2$ Hydroxy ist und $R_3$ *α-L*-Oleandrosyl ist.

6. Verfahren zur Herstellung einer Verbindung nach Anspruch 1, durch Behandeln einer Verbindung mit der Formel

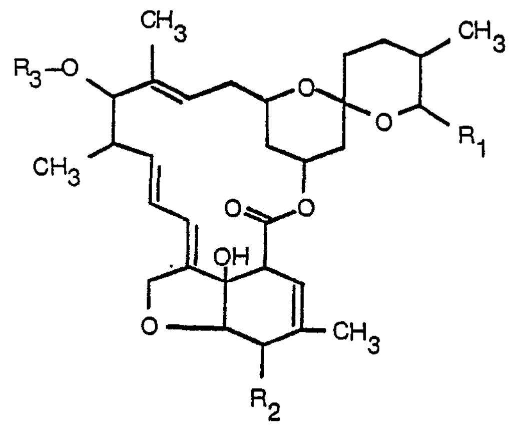

worin $R_1$, $R_2$ und $R_3$ wie in Anspruch 1 definiert sind, mit Wasserstoff in Anwesenheit einer katalytischen Menge einer Verbindung mit der Formel $[(R_4)_3P]_3RhX$, worin $R_4$ $C_{1-6}$-Alkyl, Phenyl oder der ($C_{1-6}$-Alkyl)-substituierter-Phenyl-Rest ist und X ein Halogenatom ist.

7. Verbindung nach Anspruch 1 oder ein Gemisch von zwei oder mehreren derartigen Verbindungen zur Verwendung als Parasitizid.

8. Zusammensetzung zur Behandlung von Parasiteninfektionen, die einen inerten Träger und eine oder mehrere Verbindungen nach Anspruch 1 enthält.

9. Zusammensetzung nach Anspruch 8, in der der aktive Bestandteil ein Gemisch ist aus etwa 80% 22,23-Dihydro-C—076 B1a nach Anspruch 4 und entsprechend etwa 20% 22,23-Dihydro-C—076 B1b, wobei es sich um die Verbindung nach Anspruch 1 handelt, worin $R_1$ Isopropyl ist, $R_2$ Hydroxy ist und $R_3$ Wasserstoff ist.